# EUROPEAN PATENT SPECIFICATION

(11) **EP 4 079 249 B1**
(45) Date of publication and mention of the grant of the patent: **25.10.2023**
(21) Application number: 20915931.8
(22) Date of filing: 07.04.2020
(51) Int. Cl.: A61B 34/30, A61B 34/37, A61B 34/00, A61B 90/00

(54) **OPERATING HANDLE WITH FEEDBACK OF GUIDEWIRE/CATHETER ADVANCEMENT RESISTANCE FOR VASCULAR INTERVENTION ROBOT**
HANDHABUNGSGRIFF FÜR EINEN VASKULÄREN INTERVENTIONSROBOTER MIT FÜHRUNGSDRAHTKATHETERRÜCKKOPPLUNGSWIDERSTAND
POIGNÉE DE COMMANDE DE ROBOT D'INTERVENTION VASCULAIRE AVEC RÉTROACTION DE RÉSISTANCE À L'AVANCEMENT D'UN CATHÉTER À FIL DE GUIDAGE

(30) Priority: 20.01.2020 CN 202010067542
(43) Date of publication of application: 26.10.2022
(73) Proprietor: Shanghai Operation Robot Co., Ltd., Shanghai 201321 (CN)
(72) Inventor: LI, Meng, Shanghai 201321 (CN); WANG, Kundong, Shanghai 201321 (CN); LU, Qingsheng, Shanghai 201321 (CN); LIU, Daozhi, Shanghai 201321 (CN); LIU, Yikun, Shanghai 201321 (CN); YU, Zhongwei, Shanghai 201321 (CN)
(74) Representative: Bayramoglu et al.
(86) International application number: PCT/CN2020/083457
(87) International publication number: WO 2021/147177

(56) References cited:
- WO-A1-2018/001836
- WO-A2-2009/120992
- CN-A- 105 596 084
- CN-A- 107 049 375
- CN-A- 107 184 274
- CN-A- 107 184 274
- CN-A- 108 704 214
- CN-A- 108 888 848
- CN-A- 109 199 588
- CN-A- 109 199 588
- CN-A- 110 141 366
- KR-B1- 101 949 018
- US-A1- 2011 087 238
- US-A1- 2017 000 576

## Description

### TECHNICAL FIELD

The present disclosure relates to the field of surgical robot operation, and in particular to an operating handle for a vascular intervention robot that relays the feedback of guidewire/catheter advancement resistance.

### BACKGROUND

In recent years, cardiovascular and cerebrovascular diseases have gradually become one of the main factors that threaten people's health. Minimally invasive interventional procedures have gradually become one of the main approaches for the treatment of cardiovascular diseases due to its advantages of high precision, fast speed, and small incisions. Vascular interventional procedures are performed with the aid of X-rays, which results in surgeons having long-term exposure to X-rays. To solve this problem, engineers have developed vascular intervention robots to perform minimally invasive interventional procedures on behalf of surgeons. Surgeons remotely operate vascular intervention robots in an X-ray-free environment to complete vascular interventional procedures. Currently, surgical intervention robots are controlled via touchscreens and operating handles. Vascular interventional procedures rely on a surgeon's control and feedback on the guidewire/catheter. Therefore, the operating handle must issue operating commands to the surgical robot, such as advancing/retreating and rotating the guidewire/catheter. The operating handle must also relay the resistance encountered during the advancement of the guidewire/catheter to the surgeon's hand to help the surgeon determine the current state of the guidewire/catheter in the blood vessel. The feedback of the advancement resistance of the guidewire/catheter can enhance the surgeon's sense of surgical presence, improve surgical safety, and reduce the risk of medical malpractice.

In the touchscreen control mode, the virtual keys on the touchscreen are used to control the vascular intervention robot to advance or rotate the guidewire/catheter, which is not based on the actual surgical action of the surgeon.

The Amigo^{™} Remote Catheter System, developed by Catheter Robotics, utilizes an operating handle similar to an endoscope control handle to control the motion of vascular intervention robot, including the axial and rotational movements of the guidewire/catheter and the bending angle of the catheter tip. The CorPath^{®} system, developed by Corindus Vascular Robotics, uses a joystick to control the catheter/guidewire. These two operating handles are also not based on the actual surgical action of the surgeon.

The University of Western Ontario in Canada has developed an operating handle for a vascular intervention robot based on a real guidewire/catheter system. Since the operations are performed in a way similar to the real guidewire/catheter, the operating handle can completely replicate the surgeon's hand motions during interventional surgery. However, this operating handle still cannot relay the contact force of the guidewire/catheter inside the blood vessel to the surgeon.

Many academic institutions have studied force feedback of guidewires/catheters in endovascular interventional procedures. The Shibaura Institute of Technology in Japan uses a current-controlled electrorheological fluid to feed back the resistance encountered by the guidewire/catheter in the blood vessel. In China, the Harbin Institute of Technology controls the guidewire/catheter through a roller, and the rotational resistance of the roller reflects the resistance produced by the manipulator of the robot. The Tianjin University of Technology has explored the use of a magnetorheological fluid as a force feedback medium. The Shenzhen Institute of Advanced Technology realizes the main-end operation through the force feedback that is implemented by a motor.

The existing operating handle for a vascular intervention robot mainly relies on the controller of the vascular intervention robot to issue motion instructions to the distal vascular intervention robot to control the rotation, push-pull, or compound actions of the catheter/guidewire. Although the relevant schemes and means of a vascular intervention robot are relatively well-developed, the current difficulty lies in its capability to relay the resistance encountered by the guidewire/catheter in the blood vessel to the surgeon. In particular, there is a need for an operating handle that can simulate the surgeon's real operation actions and provide force feedback. Therefore, it is necessary to explore new principles and structures in terms of real-time performance, dynamic performance, and force coupling between rotational and forward-backward translational motions.

However, there is still a lack of operating handles that replicate the operation action of the surgeon in endovascular interventional surgery and relay the movement resistance of the guidewire/catheter in the blood vessel to the surgeon's hand. The existing schemes are in the experimental stage and have the problems of high manufacturing difficulty, high technical requirements, difficult control, and high research, development, and manufacturing costs. WO2009/120992A2 discloses an input device for a robotic medical system. The input device includes a handle configured to be rotatable about a center axis, and to be longitudinally displaceable along the center axis. The input device also includes a deflection control element disposed on the handle and configured to selectively control deflection of the distal end of a flexible medical instrument electrically coupled to the input device. Longitudinal displacement of the handle may cause or result in a corresponding longitudinal motion or deflection of the flexible medical instrument. Rotation of the handle may cause or result in a corresponding rotation of the deflection plane. Longitudinal displacement and rotation of the handle may be detected or sensed electronically. CN109199588A discloses an electromagnetic damping precession force feedback operating handle for vascular intervention. The operating handle comprises an operating lever, and a frame, and also includes any one or more mechanisms of a push-pull force feedback mechanism, a rotational torque feedback mechanism, a rotational movement measuring mechanism, and non-contact reset mechanism which are mounted on the operating lever; the operating handle is supported on the frame by a left linear bearing, a right linear bearing, and the operating lever is made of ferromagnetic material. The operating handle can realize the control command issuance of push-pull, rotation and clamping as well as the resistance feedback, adopts the non-contact design except the linear bearing support. US2011/087238A1 discloses a system for performing minimally invasive cardiac procedures. CN107184274A discloses a vascular intervention surgical robot operating handle with hand feel. KR101949018B1 discloses a master device for vascular intervention. The master device comprises a rotation actuator for controlling rotation of a human body implant included in a vascular intervention slave device; a translation actuator for controlling translation of the human body implant; a handle assembly for driving the rotation actuator and the translation actuator according to an operation of an operator; and a control portion for generating a driving control signal for driving the human body implant according to the operation of the rotation actuator and the translation actuator driven by the handle assembly, and applying a haptic control signal generated according to a reaction force of the human body implant remotely controlled by the driving control signal to the rotation actuator and the translation actuator.

### SUMMARY

In order to overcome the defects in the prior art, an objective of the present disclosure is to provide an operating handle that relays the feedback of guidewire/catheter advancement resistance for a vascular intervention robot.

The operating handle that relays the feedback of guidewire/catheter advancement resistance for a vascular intervention robot provided by the present disclosure includes a sliding guide rail, a fixing base plate, a connecting rod, an operation rod, a pressure sensing device, a rotary driving device, and a linear motor.

The rotary driving device, the sliding guide rail, and a stator of the linear motor are arranged on the fixing base plate.

The pressure sensing device and a rotor of the linear motor are connected with the sliding guide rail through a slider and are able to reciprocate along the sliding guide rail.

The connecting rod has one end provided with the operation rod and the other end connected with the rotor of the linear motor through a strain gauge. The connecting rod passes through the pressure sensing device and the rotary driving device in sequence.

The rotary driving device includes a fixing base, a rotary encoder, a rotary driving piece, and a rotary driving rod.

The rotary encoder is provided on the fixing base, and the rotary driving rod is connected with a rotor of the rotary encoder.

The rotary driving piece is fixed to the connecting rod and has two ends provided with connecting holes, and the rotary driving piece is connected with the rotary driving rod through the connecting holes.

The connecting rod is able to drive the rotary driving piece to rotate, thereby driving the rotary encoder to rotate through the rotary driving rod.

The rotary driving piece is slidable on the rotary driving rod through the connecting holes.

Preferably, the pressure sensing device may include a film-type pressure sensor, a first sliding bearing, and a rotary slip ring.

The film-type pressure sensor may be wrapped on a surface of the operation rod.

The first sliding bearing and the rotary slip ring may be arranged on the sliding guide rail.

The film-type pressure sensor may be electrically connected with the rotary slip ring, and the rotary slip ring may be externally connected with a main controller.

Preferably, the operation rod further may include a second sliding bearing fixed to the fixing base plate; the first sliding bearing and the second sliding bearing support the connecting rod.

Preferably, the operating handle further may include a thrust bearing, which has one end connected with the connecting rod and the other end connected with the strain gauge.

Preferably, the linear motor may transmit a moving distance and speed of the rotor of the linear motor to the main controller through a sensor.

Preferably, the sensor may include a grating ruler, a magnetic grating ruler, a distance meter, or a printed circuit board (PCB) distance sensor.

Compared with the prior art, the present disclosure has the following beneficial effects.
1. The operating handle of the present disclosure completely replicates the actual operation of the surgeon and can be operated by the surgeon only after a short period of learning.
2. The operating handle of the present disclosure can relay the contact force of the front-end guidewire/catheter during the advancing process in real time to the surgeon's hand in the form of resistance, so as to provide the surgeon with a better sense of presence.
3. The operating handle of the present disclosure has high control precision with a linear distance resolution of 7 µm, a rotation angle resolution of 0.08°, and a force feedback resolution of 0.5 g.
4. The operating handle of the present disclosure is composed of finished parts. The operating handle is practical and can be easily manufactured at low cost.

### BRIEF DESCRIPTION OF THE DRAWINGS

Other features, objectives, and advantages of the present disclosure will become more apparent by reading the detailed description of non-limiting embodiments with reference to the following drawings.
FIGS. 1 and 2 are structural views of an operating handle for a vascular intervention robot.
FIG. 3 is a control logic for force feedback of the operating handle.

### Reference Numerals:

1. film-type strain gauge;
2. supporting sliding bearing;
3. rotary slip ring;
4. connecting rod;
5. rotary driving rod;
6. rotary driving piece;
7. rotary encoder;
8. fixing base;
9. bidirectional thrust bearing;
10. strain gauge;
11. linear motor;
12. sliding guide rail;
13. fixing base plate; and
14. operation rod.

### DETAILED DESCRIPTION OF THE EMBODIMENTS

The present disclosure is described in detail below with reference to specific embodiments. The following embodiments will help those skilled in the art to further understand the present disclosure but do not limit the present disclosure in any way. It should be noted that several variations and improvements can also be made by a person of ordinary skill in the art without departing from the concept of the present disclosure. The scope of the invention is defined by the appended claims.

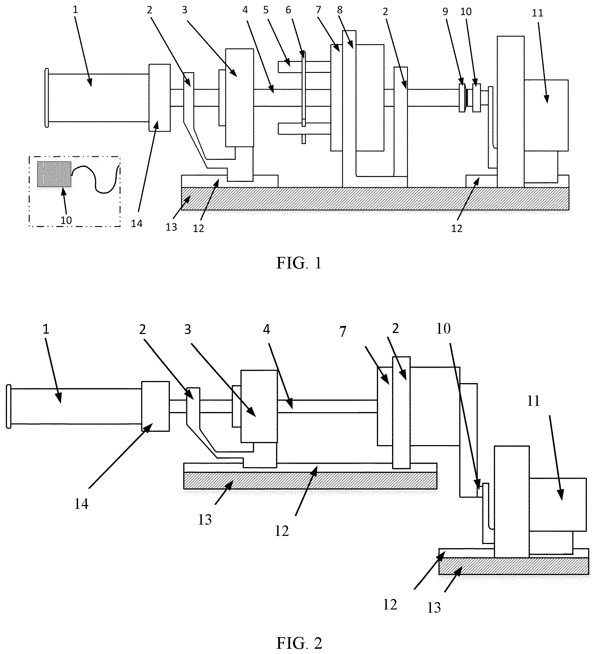

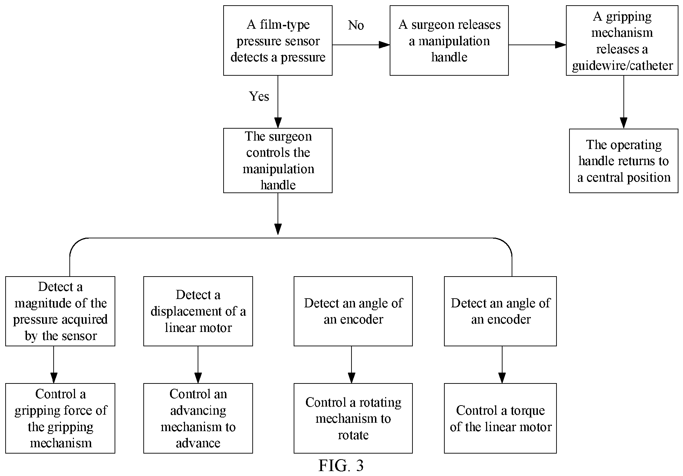

As shown in FIGS. 1 to 3, the present disclosure provides an operating handle for a vascular intervention robot that provides feedback of guidewire/catheter advancement resistance. The operating handle simulates operation actions of a surgeon in vascular interventional surgery and replicates the actions of the surgeon to advance and rotate a guidewire/catheter, so as to control the front-end intervention robot to complete gripping, advancement, and rotation of the guidewire/catheter. Since the operation actions of the surgeon include several actions, the operating handle needs to decouple the operation actions of the surgeon. A force sensor and a power actuator relay the resistance encountered by the guidewire/catheter in the blood vessel during the advancement process to the operating handle. The operating handle then transmits the resistance to the surgeon to help the surgeon determine whether the guidewire/catheter is advanced smoothly during the interventional operation. The present disclosure replicates the operation actions of the surgeon in vascular interventional surgery through a two-degree-of-freedom (2-DoF) mechanism that performs linear and rotational motions. Specifically, the operating handle includes a film-type strain gauge 1, a supporting sliding bearing 2, a rotary slip ring 3, a connecting rod 4, a rotary driving rod 5, a rotary driving piece 6, a rotary encoder 7, a fixing base 8, a bidirectional thrust bearing 9, a strain gauge 10, a linear motor 11, a sliding guide rail 12, a fixing base plate 13, and an operation rod 14. The sliding guide rail 12, a stator of the linear motor 11 and the fixing base 8 are fixed on the fixing base plate 13. The rotary slip ring 3, a rotor of the linear motor 11, and the sliding bearing 2 are connected with the sliding guide rail 12 through a slider and are movable linearly along the sliding guide rail 12. The rotary encoder 7 is hollow and fixed to the fixing base plate through the fixing base 8. The operation rod 14, the rotary driving piece 6, the bidirectional thrust bearing 9, and the strain gauge 10 are connected with the connecting rod 4. The connecting rod 4 is supported by the supporting sliding bearing 2 and is slidable linearly and rotatable in the supporting sliding bearing 2. The film-type strain gauge 1 is attached to a surface of the operation rod 14. The rotary driving piece 6 is fixed to the connecting rod 4 and has two ends provided with holes. The rotary driving piece 6 is connected with the rotary driving rod 5 through the holes and is slidable on the rotary driving rod 5 through the holes. The rotary driving rod 5 is connected with a rotor of the rotary encoder. The bidirectional thrust bearing 9 has one end connected with the connecting rod 4 and the other end connected with the strain gauge 10. The connecting rod 4 is connected with the rotor of the linear motor 11 through the strain gauge 10. Another strain gauge 10 is provided in a guidewire/catheter advancing mechanism of the distal intervention robot for measuring an advancing force applied on the guidewire/catheter during the advancing process.

In a variation of the present disclosure, the operating handle includes a film-type strain gauge 1, a supporting sliding bearing 2, a rotary slip ring 3, a connecting rod 4, a rotary encoder 7, a strain gauge 10, a linear motor 11, a fixing base 8, and a sliding guide rail 12. The sliding guide rail 12, a stator of the linear motor 11, and the fixing base 2 are fixed on the fixing base plate 13. The rotary slip ring 3, the rotary encoder 7, a rotor of the linear motor 11, and the supporting sliding bearing 2 are connected with the sliding guide rail 12 through a slider and are movable linearly along the sliding guide rail 12. The operation rod 14 and the rotary encoder 7 are connected with the connecting rod 4. The connecting rod 4 is supported by the supporting sliding bearing 2 and a rotating end of the rotary encoder 7 and is linearly slidable and rotatable in the supporting sliding bearing 2. The film-type strain gauge 1 is attached to a surface of the operation rod 14. A fixed end of the rotary encoder 7 is connected with the rotor of the linear motor 11 through the strain gauge 10.

The working principle of the present disclosure is as follows:
In endovascular interventional surgery, the surgeon needs to perform three basic actions to manipulate the guidewire/catheter: the surgeon's fingers grip/release the guidewire/catheter, the surgeon's hand advances the guidewire/catheter linearly, and the surgeon's fingers rotate the guidewire/ catheter.
I: Gripping/releasing action of the surgeon's fingers. The surgeon holds the operation rod 14 and controls a gripping force. The film-type pressure sensor 1 wrapped on the surface of the operation rod 14 converts the surgeon's gripping force into a current signal and transmits the current signal to a main controller through the rotary slip ring 3. The main controller identifies the surgeon's gripping action based on the current signal transmitted by the film-type pressure sensor 1. When the film-type pressure sensor 1 detects a pressure, it indicates that the surgeon is performing a gripping action. Thus, a gripping mechanism of the distal intervention robot is controlled to grip the guidewire/catheter. When the film-type pressure sensor 1 detects no pressure, it indicates that the surgeon is performing a releasing action. Thus, the gripping mechanism of the distal intervention robot is controlled to release the guidewire/catheter. The film-type pressure sensor 1 detects the magnitude of the surgeon's gripping force through a magnitude of an analog signal, thereby adjusting the magnitude of the gripping force of the gripping mechanism of the distal intervention robot.
II: Linear advancing action of the surgeon's hand. The surgeon controls the guidewire/catheter to advance linearly by linearly pulling or pushing the connecting rod 4 by the operation rod 14. The connecting rod 4 drives the rotor of the linear motor 11 to move through the bidirectional thrust bearing 9. A ruler (or a magnetic grating ruler, a distance meter, or a printed circuit board (PCB) distance sensor) of the linear motor 11 transmits a moving distance and speed of the rotor to the main controller. The moving distance and speed of the rotor reflect the pushing distance and speed of the surgeon's hand, and the distance and speed of the guidewire/catheter advanced by an advancing mechanism of the distal intervention robot are controlled based on the moving distance and speed of the rotor.
III: Rotating action of the surgeon. The surgeon rotates the connecting rod 4 by rotating the operation rod 14, thereby controlling the rotation of the guidewire/catheter. The rotation of the connecting rod 4 drives the rotary driving piece 6 to rotate, thereby driving the rotary driving rod 5 to rotate. The rotation of the rotary driving rod 5 drives the rotary encoder 7 to rotate. The rotary encoder acquires and transmits the angle and speed of the surgeon's rotating action to the main controller, so as to control the angle and speed of a rotating mechanism of the distal intervention robot to rotate the guidewire/catheter.

The advancing mechanism of the distal intervention robot is further provided therein with a strain gauge. The strain gauge detects the resistance of the guidewire/catheter during the advancing process, and transmits the resistance to the main controller in the form of an electrical signal. The main controller adjusts the current of the linear motor 11 according to the front-end resistance to generate a corresponding push or pull force until the current value acquired by the strain gauge 10 is equal to the current value measured by a front-end strain gauge. Since the surgeon is controlling the operation rod 14, the push/pull force of the linear motor is relayed to the surgeon through the connecting rod. As a result, the surgeon's hand experiences a resistance similar to that encountered in a real scenario of physically advancing the guidewire/catheter.

In the present disclosure, the linear motor 11 is configured to measure the operating displacement and transmit the resistance of the guidewire/catheter. The linear motor 11 measures the pushing distance of the surgeon by transmitting its position. The linear motor drives the operation rod to automatically return to a central position through its position control mode and relays the advancing force to the surgeon through its torque control mode. The present disclosure solves the problems that the multi-DoF motion of the decoupling mechanism of the rotary slip ring 3, the crank-like rocker mechanism, and the direct thrust bearing (or magnet) conflicts with the single-DoF motion of a single component and that the cords may become tangled. The present disclosure decouples the movement of the gripping force sensor and the restraint of the fixed cord through the hollow rotary slip ring 3. The gripping force sensor is fixedly connected with the operation rod and is movable linearly and rotationally with the operation rod, and the lead wires of the gripping force sensor are also movable with the operation rod. The present disclosure solves the problem of cord tangling by decoupling the rotational motion and the linear motion through the hollow rotary slip ring 3. The present disclosure decouples the movement of the operation rod and the rotational movement of the rotary encoder 7 through the sliding bearing and the crank structure. The connecting rod 4, the rotary driving piece 6 and the rotary driving rod 5 form the crank structure, which can measure the rotation angle and speed of the connecting rod 4 without disturbing the linear motion of the connecting rod 4. The present disclosure decouples the rotational motion of the operation rod and the linear motion of the linear motor 11 through the bidirectional thrust bearing 9 or a magnet. The connecting rod 4 can perform linear motion and rotational motion, that is, 2-DOF motion, while the linear motor 11 can only perform linear motion. Therefore, the present disclosure decouples the 2-DOF motion of the connecting rod 4 and the single-DOF motion of the linear motor 11 through the bidirectional thrust bearing 9 or the magnet.

The specific embodiments of the present disclosure are described above. It should be understood that the present disclosure is not limited to the above specific implementations, and a person skilled in the art can make variations or modifications within the scope of the claims The embodiments in the present disclosure and features in the embodiments may be combined with each other in a nonconflicting situation.

## Claims

1. An operating handle with a feedback of guidewire/catheter advancement resistance for a vascular intervention robot comprising a sliding guide rail (12), a fixing base plate (13), a connecting rod (4), an operation rod (14), a rotary driving device, and a motor, wherein a rotor of the motor is connected with the sliding guide rail (12) through a slider; the connecting rod has a first end and a second end, the first end is provided with the operation rod (14); the connecting rod passes through the rotary driving device;
the rotary driving device, the sliding guide rail, and a stator of the motor are arranged on the fixing base plate; the rotary driving device comprises a fixing base, a rotary encoder, and a rotary driving piece, the rotary encoder is provided on the fixing base;
**characterized in that** the operating handle further comprises a pressure sensing device, the motor is a linear motor (11);
the pressure sensing device is connected with the sliding guide rail (12) through the slider; the pressure sensitive device and a rotor of the linear motor are configured to reciprocate along the sliding guide rail;
the second end of the connecting rod is connected with the rotor of the linear motor through a strain gauge (10); and the connecting rod passes through the pressure sensing device and the rotary driving device in sequence;
the rotary driving device further comprises a rotary driving rod (5), and the rotary driving rod (5) is connected with a rotor of the rotary encoder (7); the rotary driving piece (6) is fixed to the connecting rod (4) and has two ends provided with connecting holes; and the rotary driving piece (6) is connected with the rotary driving rod (5) through the connecting holes; and the connecting rod (4) is configured to drive the rotary driving piece (6) to rotate so as to drive the rotary encoder (7) to rotate through the rotary driving rod (5); wherein the rotary driving piece (6) is slidable on the rotary driving rod (5) through the connecting holes.

2. The operating handle according to claim 1, wherein the pressure sensing device comprises a film-type pressure sensor (1), a first sliding bearing, and a rotary slip ring (3),
the film-type pressure sensor is wrapped on a surface of the operation rod;
the first sliding bearing and the rotary slip ring are arranged on the sliding guide rail; and
the film-type pressure sensor is electrically connected with the rotary slip ring, and the rotary slip ring is externally connected with a main controller.

3. The operating handle according to claim 2, further comprising a second sliding bearing, wherein the second sliding bearing is fixed to the fixing base plate, and the first sliding bearing and the second sliding bearing support the connecting rod.

4. The operating handle according to claim 1, further comprising a thrust bearing (9), wherein a first end of the thrust bearing is connected with the connecting rod and a second end of the thrust bearing is connected with the strain gauge.

5. The operating handle according to claim 1, wherein the linear motor is configured to transmit a moving distance and speed of the rotor of the linear motor to a main controller through a sensor.

6. The operating handle according to claim 5, wherein the sensor comprises a grating ruler, a magnetic grating ruler, a distance meter, or a printed circuit board (PCB) distance sensor.

## Patentansprüche

1. Bediengriff mit Rückkopplung eines Widerstands beim Vorschieben eines Führungsdrahts/Katheters für einen Roboter für vaskuläre Eingriffe, umfassend eine Führungsschiene (12), eine feste Grundplatte (13), eine Verbindungsstange (4), einen Bedienknopf (14), eine Drehantriebsvorrichtung und einen Motor, wobei ein Rotor des Motors über einen Schieber mit der Führungsschiene (12) verbunden ist; der Verbindungsstab ein erstes und ein zweites Ende aufweist, das erste Ende mit dem Bedienknopf (14) versehen ist; die Verbindungsstange durch die Drehantriebsvorrichtung verläuft;
die Drehantriebsvorrichtung, die Führungsschiene und ein Stator des Motors auf der festen Grundplatte angeordnet sind; die Drehantriebsvorrichtung einen festen Sitz, einen Drehgeber und ein Drehantriebsstück aufweist, wobei der Drehgeber auf dem festen Sitz bereitgestellt ist;
**dadurch gekennzeichnet, dass** der Bediengriff ferner eine Druckmessvorrichtung aufweist, der Motor ein Linearmotor (11) ist;
die Druckmessvorrichtung über den Schieber mit der Führungsschiene (12) verbunden ist; die Druckmessvorrichtung und ein Rotor des Linearmotors dazu eingerichtet sind, sich entlang der Führungsschiene hin- und herzubewegen;
das zweite Ende der Verbindungsstange über einen Dehnungsmesser (10) mit dem Rotor des Linearmotors verbunden ist; und die Verbindungsstange nacheinander durch die Druckmessvorrichtung und die Drehantriebsvorrichtung verläuft;
wobei die Drehantriebsvorrichtung ferner eine Drehantriebsstange (5) aufweist und die Drehantriebsstange (5) mit einem Rotor des Drehgebers (7) verbunden ist; das Drehantriebsstück (6) an der Verbindungsstange (4) fixiert ist und zwei Enden aufweist, die mit Verbindungslöchern versehen sind; und das Drehantriebsstück (6) durch die Verbindungslöcher mit der Drehantriebsstange (5) verbunden ist; und die Verbindungsstange (4) dazu eingerichtet ist, das Drehantriebsstück (6) zu Drehungen anzutreiben, wodurch der Drehgeber (7) zu Drehungen durch die Drehantriebsstange (5) angetrieben wird; wobei das Drehantriebsstück (6) auf der Drehantriebsstange (5) durch die Verbindungslöcher schiebbar ist.

2. Bediengriff nach Anspruch 1, wobei die Druckmessvorrichtung einen Dünnfilm-Drucksensor (1), ein erstes Gleitlager und einen Schleifring (3) umfasst,
der Dünnfilm-Drucksensor um eine Fläche des Bedienknopfs gewickelt ist;
das erste Gleitlager und der Schleifring auf der Führungsschiene angeordnet sind; und
der Dünnfilm-Drucksensor elektrisch mit dem Schleifring verbunden ist und der Schleifring extern mit einer Hauptsteuervorrichtung verbunden ist.

3. Bediengriff nach Anspruch 2, ferner umfassend ein zweites Gleitlager, wobei das zweite Gleitlager an der festen Grundplatte fixiert ist und das erste Gleitlager und das zweite Gleitlager die Verbindungsstange stützen.

4. Bediengriff nach Anspruch 1, ferner umfassend ein Drucklager (9), wobei ein erstes Ende des Drucklagers mit der Verbindungsstange verbunden ist und ein zweites Ende des Drucklagers mit dem Dehnungsmesser verbunden ist.

5. Bediengriff nach Anspruch 1, wobei der Linearmotor dazu eingerichtet ist, über einen Sensor eine Bewegungsdistanz und eine Geschwindigkeit des Rotors des Linearmotors an eine Hauptsteuervorrichtung zu übertragen.

6. Bediengriff nach Anspruch 5, wobei der Sensor ein Gittermessgerät, ein magnetisches Gittermessgerät, ein Distanzmessgerät oder einen Leiterplatten-Distanzsensor umfasst.

## Revendications

1. Poignée de commande avec rétroaction de résistance à l'avancement d'un fil guide/cathéter pour un robot d'intervention vasculaire comprenant un rail de guidage coulissant (12), une plaque de base de fixation (13), une bielle (4), une tige de commande (14), un dispositif d'entraînement rotatif et un moteur, un rotor du moteur étant relié au rail de guidage coulissant (12) par l'intermédiaire d'un curseur ; la bielle présentant une première extrémité et une deuxième extrémité, la première extrémité étant pourvue de la tige de commande (14) ; la bielle passant à travers le dispositif d'entraînement rotatif ;
le dispositif d'entraînement rotatif, le rail de guidage coulissant et un stator du moteur linéaire étant disposés sur la plaque de base de fixation ; le dispositif d'entraînement rotatif comprenant une base de fixation, un codeur rotatif, et une pièce d'entraînement rotative, le codeur rotatif étant fourni sur la base de fixation ;
**caractérisée en ce que** la poignée de commande comprend en outre un dispositif de détection de pression, le moteur est un moteur linéaire (11) ;
le dispositif de détection de pression est relié au rail de guidage coulissant (12) par l'intermédiaire du curseur ; le dispositif de détection de pression et un rotor du moteur linéaire sont configurés pour effectuer un mouvement de va-et-vient le long du rail de guidage coulissant ;
la deuxième extrémité de la bielle est reliée au rotor du moteur linéaire par l'intermédiaire d'une jauge de contrainte (10) ; et la bielle passe séquentiellement à travers le dispositif de détection de pression et le dispositif d'entraînement rotatif ;
le dispositif d'entraînement rotatif comprend en outre une tige d'entraînement rotative (5), et la tige d'entraînement rotative (5) est reliée à un rotor du codeur rotatif (7) ; la pièce d'entraînement rotative (6) est fixée à la bielle (4) et présente deux extrémités pourvues de trous de liaison ; et la pièce d'entraînement rotative (6) est reliée à la tige d'entraînement rotative (5) par l'intermédiaire des trous de liaison ; et la bielle (4) est configurée pour entraîner la pièce d'entraînement rotative (6) en rotation afin d'entraîner le codeur rotatif (7) en rotation par l'intermédiaire de la tige d'entraînement rotative (5) ; la pièce d'entraînement rotative (6) pouvant coulisser sur la tige d'entraînement rotative (5) à travers les trous de liaison.

2. Poignée de commande selon la revendication 1, dans laquelle le dispositif de détection de pression comprend un capteur de pression de type film (1), un premier palier lisse et un collecteur tournant (3),
le capteur de pression de type film est enveloppé sur une surface de la tige de commande
le premier palier lisse et le collecteur tournant sont disposés sur le rail de guidage coulissant ; et
le capteur de pression de type film est relié électriquement au collecteur tournant, et le collecteur tournant est relié extérieurement à un contrôleur principal.

3. Poignée de commande selon la revendication 2, comprenant en outre un deuxième palier lisse, dans laquelle le deuxième palier lisse est fixé à la plaque de base de fixation, et le premier palier lisse et le deuxième palier lisse supportent la bielle.

4. Poignée de commande selon la revendication 1, comprenant en outre un palier de butée (9), dans laquelle une première extrémité du palier de butée est reliée à la bielle et une deuxième extrémité du palier de butée est reliée à la jauge de contrainte.

5. Poignée de commande selon la revendication 1, dans laquelle le moteur linéaire est configuré pour transmettre une distance de déplacement et une vitesse du rotor du moteur linéaire au contrôleur principal par l'intermédiaire d'un capteur.

6. Poignée de commande selon la revendication 5, dans laquelle le capteur comprend une règle de réseau, une règle de réseau magnétique, un télémètre ou un capteur de distance sur carte de circuit imprimé (PCB).
